# EUROPEAN PATENT SPECIFICATION

(11) **EP 2 152 253 B1**
(45) Date of publication and mention of the grant of the patent: **18.04.2018**
(21) Application number: 07728789.4
(22) Date of filing: 04.05.2007
(51) Int. Cl.: G01N 33/00, A61K 31/047, A61K 45/06, A23L 3/349, A61K 8/34, C11D 3/48, C11D 3/20, A61Q 17/00, A61Q 19/00

(54) **SYNERGISTIC ACTIVE PREPARATIONS COMPRISING 1,2-DECANEDIOL AND FURTHER ANTIMICROBIAL ACTIVE COMPOUNDS**
SYNERGISTISCHE WIRKSAME ZUBEREITUNGEN MIT 1,2-DECANDIOL UND WEITERE ANTIMIKROBIELLE WIRKVERBINDUNGEN
COMPOSITIONS À ACTIVITÉ SYNERGIQUE COMPRENANT DU 1,2-DÉCANÉDIOL ET D'AUTRES CPMPOSÉS ANTIMICROBIENS ACTIFS

(43) Date of publication of application: 17.02.2010
(73) Proprietor: Symrise AG, 37603 Holzminden (DE)
(72) Inventor: SCHMAUS, Gerhard, 37671 Höxter (DE); PILLAI, Ravikumar, Emerson, NJ 07630 (US)
(74) Representative: Fabry, Bernd
(86) International application number: PCT/EP2007/054336
(87) International publication number: WO 2008/135085

(56) References cited:
- EP-A- 1 297 829
- EP-A- 1 598 064
- DATABASE WPI Week 200505 Derwent Publications Ltd., London, GB; AN 2005-042854 XP002441411 -& JP 2004 352688 A (KAO CORP) 16 December 2004 (2004-12-16)
- Anonymous: "DISINFECTANTS FEDERALLY-APPROVED FOR USE AGAINST VIRUSES OF HIGHLY CONTAGIOUS DISEASES", , 12 May 2006 (2006-05-12), XP55019430, INTERNET Retrieved from the Internet: URL:http://www.michigan.gov/documents/MDA_ USDA_Approved_Disinfectants_HCD_2Dec05_151 926_7.pdf [retrieved on 2012-02-15] & ANONYMOUS: "Approved Disinfectants HCD", INTERNET CITATION, 12 May 2006 (2006-05-12), page 1, XP007920255, Retrieved from the Internet: URL:http://www.michigan.gov/documents/MDA_ USDA_Approved_disinfectants_HCD _2Dec05_151926_7.pdf> [retrieved on 2012-02-15]
- ALAN D RUSSELL ED - BLOCK S S: "Principles of Antimicrobia Activity and REsistance", DISINFECTION, STERILIZATION, AND PRESERVATION, LIPPINCOTT [U.A.], PHILADELPHIA, PA. [U.A.], vol. Chapter 3, 15 December 2000 (2000-12-15), pages 31-55, XP009156531, ISBN: 978-0-683-30740-5

## Description

The present invention relates to specific synergistic antimicrobial (cosmetic or pharmaceutical) preparations comprising a mixture comprising or consisting of
a) an antimicrobial active amount of 1,2-decanediol of the formula 1: 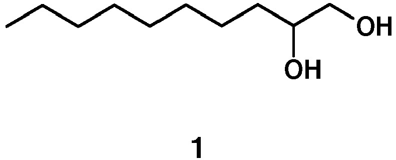 and
b) an antimicrobial active amount of one or more compounds selected from the group consisting of ethanol, propan-1-ol, propan-2-ol; chlorhexidine digluconate, chloroxylenol, triclosan, triclocarban, benzethonium chloride, methylbenzethonium chloride and benzalkonium chloride,
   wherein the preparation additionally comprises or consists of
c) an antimicrobial active amount of one or more compounds selected from the group consisting of mecetroniumetil sulfate, undecyleneamidopropyltrimonium methosulfate, (ethylendioxy)dimethanol, benzyl-C12-18-alkyldimethylammoniumchloride, didecyldimethylammonium chloride, N,N-didecyl-N-methyl-poly(oxethyl)ammonium propionate, N-(3-aminopropyl)-N-dodecylpropan-1,3-diamin, N-dodecylpropan-1,3-diamin, N-(3-aminopropyl)-N-dodecylpropan-1,3-diamin, clorofen, 2-biphenyl-2-ol, chlorocresol, hydrogen peroxide, acetic acid, peracetic acid, glutaral and formaldehyde,
wherein the total amount of 1,2-decanediol of formula 1 in the preparation is in the range of from 0.1 to 2 wt.-% based on the weight of the preparation.

In the field of the cosmetics and pharmaceutical industry and household products industry, there is an increasing demand for antimicrobial agents especially for hygiene products like antimicrobial liquid or solid soaps, disinfectant cleansing solutions for the treatment of skin, specifically for the treatment of hands and/or for the treatment of technical surfaces like e.g. surgery equipment.

Quite commonly used antimcirobial active materials frequently used in such type of products are, e.g. ethanol, propan-1-ol, propan-2-ol or quaternary antimicrobial agents such as benzethonium chloride, methylbenzethonium chloride or benzalkonium chloride. However, the use of high concentrations of such type of antimicrobial actives may cause problems specifically when applied on skin. High concentrations of ethanol, propan-1-ol or propan-2-ol significantly dries out skin or may irritate skin. Furtheron alcohols with low boiling point have no residual antimicrobial activity. Higher concentrations of quaternary compounds like benzethonium chloride, methylbenzethonium chloride or benzalkonium chloride cause tackiness and stickiness resulting in an unpleasant feel of cosmetic and dermatolgical formulations on skin. Furtheron, many commonly used antimicrobial agents possess insufficient antimicrobial activity when used alone in hygiene products for sanitization of skin and technical surfaces, because of their lack of broad spectrum activity.

In the search for novel agents which antimicrobial action for use in hygiene products for disinfection of skin and technical surfaces, like e.g. antimicrobial liquid and solid soaps or antimicrobial cleansing solutions, efforts are accordingly being made quite generally to discover new substance combinations which inhibit the individual microorganisms at the lowest possible concentration whereby it is furthermore required to be ensured that these mixtures used in cosmetic and/or pharmaceutical and/or household products, in addition to having a high activity at the lowest possible concentrations, must also additionally be
- toxicologically acceptable,
- readily tolerated by the skin, in the sense that they do not cause skin dryness, skin irritation or unpleasant skin feel like tackiness or stickiness
- heat-stable (in particular in the conventional cosmetic and/or pharmaceutical preparations),
- preferably odourless and
- inexpensive to prepare (i.e. employing standard processes and/or starting from standard precursors).

Therefore there is a constant need for new highly efficient antimicrobial compositions in a format convenient for desinfecting hygiene products like liquid and solid soaps and alcohol, water- and/or glycol based cleansing solutions which do not dry skin, do not irritate skin, leave the skin smooth, comfortable and adequate moisturized

Mosturizing properties of aliphatic 1,2-alkanediols with carbon chain length C5 to C10 are disclosed in EP 0 655 904. As further disclosed in EP 1 269 983 and DE 103 41 179, 1,2-decanediol also shows antimicrobial activity against microorganisms causing body odour (Staphylococcus epidermidis, Corynebacterium acnes) and acne (Propionibacterium acnes). Because of its moistrizing and antimicrobial activity 1,2-decanediol is already used in cosmetic or dermatological formulations as moisturizer and to treat body odour and acne. However these patent applications do not describe the use of 1,2-decanediol in combination with one or more compounds selected from the group consisting of ethanol (CARN 64-17-5; INCI name: Alcohol), propan-1-ol (CARN 71-23-8; INCI name: Propyl Alcohol), propan-2-ol (CARN 67-63-0; INCI name: Isopropyl Alcohol), chlorhexidine digluconate (CARN 18472-51-0; INCI name: Chlorhexidine Digluconate), chloroxylenol (CARN 88-04-0; 1321-23-9; INCI name: Chloroxylenol), triclosan (CARN 3380-34-5; INCI name: Triclosan), triclocarban (CARN 101-20-2; 1322-40-3; INCI name: Triclocarban), benzethonium chloride (CARN 121-54-0; INCI name: Benzethonium Chloride), methylbenzethonium chloride (CARN 25155-18-4; INCI name: Methylbenzethonium Chloride) and benzalkonium chloride (CARN 8001-54-5, 61789-71-7, 68391-01-5, 68424-85-1 , 85409-22-9, INCI name: Benzalkonium Chloride) in hygiene products like disinfectant liquid or solid soaps and disinfectant cleansing solutions for the treatment of skin, specifically for the treatment of hands and/or for the treatment of technical surfaces like surgery equipment or technical surfaces in general.

EP 0 524 548 discloses the use of aliphatic 1,2-diols with carbon chain length C8 to C24 for the preparation of disinfecting cleansing solutions for skin. However, the use of 1,2-decanediol in combination with one or more compounds selected from the group consisting of ethanol (CARN 64-17-5; INCI name: Alcohol), propan-1-ol (CARN 71-23-8; INCI name: Propyl Alcohol), propan-2-ol (CARN 67-63-0; INCI name: Isopropyl Alcohol), chlorhexidine digluconate (CARN 18472-51-0; INCI name: Chlorhexidine Digluconate), chloroxylenol (CARN 88-04-0; 1321-23-9; INCI name: Chloroxylenol), triclosan (CARN 3380-34-5; INCI name: Triclosan), triclocarban (CARN 101-20-2; 1322-40-3; INCI name: Triclocarban), benzethonium chloride (CARN 121-54-0; INCI name: Benzethonium Chloride), methylbenzethonium chloride (CARN 25155-18-4; INCI name: Methylbenzethonium Chloride) and benzalkonium chloride (CARN 8001-54-5, 61789-71-7, 68391-01-5, 68424-85-1 , 85409-22-9, INCI name: Benzalkonium Chloride) in hygiene products like antimicrobial liquid or solid soaps, disinfectant cleansing solutions for the treatment of skin, specifically for the treatment of hands and/or for the treatment of technical surfaces like surgery is not disclosed.

The application EP 1 297 829 A discloses that an active agent combination comprising at least one polyol, preferably diols, and at least one 2,2-dialkylacetic acid can be used in antimicrobial cosmetic or dermatological compositions.

The Japanese application JP 2004 352688 A provides an antiseptic agent composition comprising an antiseptic agent and 1,2-decanediol. The antiseptic power of the antiseptic agent can be kept even the used amount is decreased.

In the search for new and improved methods for disinfection of skin and for the treatment of any type of technical surfaces the aim of a person skilled in the art is on the one hand to find new combinations of antimicrobial actives that are effective already at lower concentration to avoid any undesirable effects caused by high dosages of said single components. On the other hand it is conceivable to reduce the concentration of the commercially available agents with the undesirable side effects and combine these agents with other agents, which show less side effects and are more compatible. A synergistic combination of a commercially available agent with side effects with one or more other agents with less side effect would be ideal.

Thus, surprisingly a cosmetic, pharmaceutical and/or household product preparation, comprising a mixture comprising or consisting of
a) an antimicrobial active amount of 1,2-decanediol of formula 1: 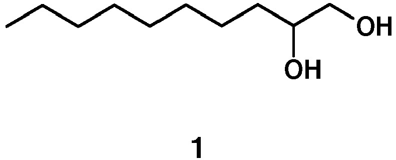 and
b) an antimicrobial active amount of one or more compounds selected from the group consisting of ethanol, propan-1-ol, propan-2-ol, chlorhexidine digluconate, chloroxylenol, triclosan, triclocarban, benzethonium chloride, methylbenzethonium chloride and benzalkonium chloride,
   wherein the preparation additionally comprises or consists of
c) an antimicrobial active amount of one or more compounds selected from the group consisting of mecetroniumetil sulfate, undecyleneamidopropyltrimonium methosulfate, (ethylendioxy)dimethanol, benzyl-C12-18-alkyldimethylammoniumchloride, didecyldimethylammonium chloride, N,N-didecyl-N-methyl-poly(oxethyl)ammonium propionate, N-(3-aminopropyl)-N-dodecylpropan-1,3-diamin, N-dodecylpropan-1,3-diamin, N-(3-aminopropyl)-N-dodecylpropan-1,3-diamin, clorofen, 2-biphenyl-2-ol, chlorocresol, hydrogen peroxide, acetic acid, peracetic acid, glutaral and formaldehyde,
wherein the total amount of 1,2-decanediol of formula 1 in the preparation is in the range of from 0.1 to 2 wt.-% based on the weight of the preparation,
fulfils the requirements of the present invention, as the preparation according to the invention comprises a synergistic combination of the antimicrobial agent of constituent a) and one or more agents of constituent b) and additionally one or more agents of constituent c).

Another embodiment of the present invention relates to a use of the preparation according to the present invention as antimicrobial agents in hygiene products like disinfectant liquid and solid soaps, disinfectant cleansing solutions and disinfectant emulsions for treatment of skin surfaces, in particular as antimicrobial preparation for technical surfaces.

A still further embodiment of the present invention relates to a pharmaceutical product preparation according to the present invention for use in a method for disinfection of skin, preferably for disinfection of hand surface, comprising or consisting of the step:
a) application of a pharmaceutical product preparation according to the present invention to skin surface, preferably to hand surface.

A still further embodiment of the present invention relates to a method for disinfection of technical surfaces, comprising or consisting of the step:
a) application of a preparation according to the present invention to technical surfaces.

A yet still further embodiment of the present invention relates to a process for the production of a preparation according to the present invention, in particular a cosmetic and/or pharmaceutical preparation for disinfection of skin, preferably for disinfection of hand surface, and/or household product preparation for disinfection of technical surfaces comprising or consisting of the following steps:
a) providing 1,2-decanediol of formula 1: 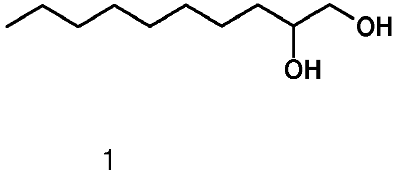
b) providing one or more compounds selected from the group of ethanol, propan-1-ol, propan-2-ol, chlorhexidine digluconate, chloroxylenol, triclosan, triclocarban, benzethonium chloride, methylbenzethonium chloride and benzalkonium chloride,
c) providing one or more compounds selected from the group consisting of mecetroniumetil sulfate, undecyleneamidopropyltrimonium methosulfate, (ethylendioxy)dimethanol, benzyl-C12-18-alkyldimethylammoniumchloride, didecyldimethylammonium chloride, N,N-didecyl-N-methyl-poly(oxethyl)ammonium propionate, N-(3-aminopropyl)-N-dodecylpropan-1,3-diamin, N-dodecylpropan-1,3-diamin, N-(3-aminopropyl)-N-dodecylpropan-1,3-diamin, clorofen, 2-biphenyl-2-ol, chlorocresol, hydrogen peroxide, acetic acid, peracetic acid, glutaral and formaldehyde, and
d) mixing one or more compounds provided in step d) with 1,2-decanediol provided in step a) and one or more compounds provided in step b) to form a preparation according to the present invention.

The following preferred aspects are relevant for each embodiment of the present invention and all combinations thereof are disclosed herewith.

Preferred embodiments of the preparations which are preferred according to the invention and uses thereof are described in the following and in the examples and the claims. The preferred use level ranges and use level ratios of different antimicrobial agents used in combination with 1,2-decanediol of the preparations with synergistic activity according to the invention are further shown in table 1.

**Table 1: Preferred use level ranges and use level ratios of different types of antimicrobial agents used in combination with 1,2-decanediol (with preferred use level of 0.1% to 2%) in finished cosmetic, pharmaceutical and household product preparations, in particular dermatological hygiene preparations for skin disinfection, specifically for hand disinfection and/or for disinfection of technical surfaces.**

| **Antimicrobial Agents** | use level range [weight %] | use level ratio antimicrobial agent: 1,2-decanediol | preferred use level ratio antimicrobial agent: 1,2-decanediol |
|---|---|---|---|
| ethanol | 50.0 - 98.0 | 25 : 1 to 980 : 1 | 50 : 1 to 500 : 1 |
| propyl alcohol | 50.0 - 98.0 | 25 : 1 to 980 : 1 | 50 : 1 to 500 : 1 |
| Isopropyl alcohol | 50.0 - 98.0 | 25 : 1 to 980 : 1 | 50 : 1 to 500 : 1 |
| triclosan | 0.1 - 1 | 1 : 20 to 10 : 1 | 1 : 10 to 5 : 1 |
| triclocarban | 0.1 - 2 | 1 : 20 to 20 : 1 | 1 : 10 to 10 : 1 |
| chlorohexidine digluconate | 0.1 - 4 | 1 : 20 to 40 : 1 | 1 : 10 to 20 : 1 |
| chloroxylenol | 0.2 - 4 | 1 :10 to 40 : 1 | 1 : 5 to 20 : 1 |
| benzethonium chloride | 0.05 - 0.5 | 1 : 40 to 5 : 1 | 1 : 20 to 2 : 1 |
| methylbenzethoniu m chloride | 0.02 - 1.0 | 1 : 100 to 10 : 1 | 1 : 50 to 5 : 1 |
| benzalkonium chloride | 0.01 - 20 | 1 : 20 to 200 : 1 | 1 : 10 to 50 : 1 |

In this context of the present invention the inventors have shown that the common antimicrobial agents used as compounds in constituent b), which are in particular disclosed in table 1, show an skin disinfecting property. When 1,2-decanediol is combined with one or more compounds of constituent b) the inventors could show a synergistic improved disinfecting activity of the resulting preparation, in particular when used in cosmetic, dermatological products for skin disinfection, preferably for hand disinfection, and/or in household products for disinfection of technical surfaces.

The inventors could show that the strongest synergistic antimicrobial effects could be received when 1,2-decanediol of the formula 1 is present in the preparation according to the invention at a concentration from 0,1 to 2 wt% based on the weight of the finished preparation.

The reason for the synergistic activity between 1,2-decanediol and compounds selected from the group selected of
ethanol (CARN 64-17-5; INCI name: Alcohol), propan-1-ol (CARN 71-23-8; INCI name: Propyl Alcohol), propan-2-ol (CARN 67-63-0; INCI name: Isopropyl Alcohol), chlorhexidine digluconate (CARN 18472-51-0; INCI name: Chlorhexidine Digluconate), chloroxylenol (CARN 88-04-0; 1321-23-9; INCI name: Chloroxylenol), triclosan (CARN 3380-34-5; INCI name: Triclosan), triclocarban (CARN 101-20-2; 1322-40-3; INCI name: Triclocarban), benzethonium chloride (CARN 121-54-0; INCI name: Benzethonium Chloride), methylbenzethonium chloride (CARN 25155-18-4; INCI name: Methylbenzethonium Chloride) and benzalkonium chloride (CARN 8001-54-5, 61789-71-7, 68391-01-5, 68424-85-1, 85409-22-9, INCI name: Benzalkonium Chloride)
has not clearly been identified yet. It may be of very different origins, whereby it seems that some mechanisms may still not have been identified yet. However, at the moment the following explanations seem plausible:
- 1,2-decanediol acts as a penetration enhancer thus helping one or more antimicrobial agents of constituent b) to better reach their biological target, either the cell wall, the cell membrane or in the cytosol of the microorganism,
- 1,2-decanediol shows sebum fluidizing properties which also helps to get in close contact with the target microorganism especially located in the skin surface sebum,
- 1,2-decanediol acts as solubilizer at least for more polar antimicrobial agents of constituent b) thus avoiding complete rinse off of compounds of constituent b) during the washing process,
however, the explanations given above are at the moment rather a mere speculative reasoning.

The particularly suitable preparations with synergistic activity according to the invention are chiefly used according to the invention for cosmetic and dermatological reasons, but they can also be provided in household products used for the disinfection of technical surfaces like surgery equipment and technical surfaces in general.

In this context, the concentration of 1,2-decanediol in the finished preparation according to the invention, in particular to be applied topically on skin, specifically on hands, and/or on technical surfaces, is in the range of from 0.1 to 2 wt.%. The antimicrobial active compound can be employed here (a) prophylactically or (b) as required.

The concentration of the amount of active compound to be applied e.g. daily varies and depends on the physiological state of the subject and individual-specific parameters, such as age or body weight.

It is to be pointed out that the term 1,2-decanediol in the context of the present invention also includes the pure S-configured enantiomer (CARN: 84276-14-2), the R-configured enantiomer (CARN: 87827-60-9) and any desired mixtures of Sand R-configured enantiomers. For commercial reasons, it is indeed particularly advantageous in these cases to employ mixtures of racemates of 1,2-decanediol as antimicrobial agent, since these are particularly readily accessible by synthesis, but the pure enantiomers or non-racemic mixtures of these enantiomers are likewise suitable for the purposes according to the invention.

The cosmetic, pharmaceutical, in particular dermatological preparations, or household product preparations according to the invention are such as, inter alia, liquid and solid soap, aqueous, ethanolic and glycolic disinfection solutions, pump sprays, aerosol sprays, creams, ointments, tinctures, lotions and specific nail care products and the like.

The cosmetic and/or pharmaceutical, in particular dermatological preparations for disinfection of skin, specifically disinfection of hands and/or household product preparations for disinfection of technical surfaces according to the invention comprising the mixture comprising or consisting of a) 1,2-decanediol and one or more compounds from constituent b) and additionally one or more compounds selected from constituent c), wherein the total amount of 1,2-decanediol in the preparation is in the range of from 0.1 to 2 wt.-% based on the weight of the preparation, including the preferred embodiments described herein, can additionally comprise conventional auxiliary compounds and additives (base ingredients) and serve for the treatment of skin and/or hair in the sense of a pharmaceutical, in particular dermatological treatment or a treatment in the sense of antimicrobial cosmetics and dermatological products and in the sense of disinfection of technical surfaces for household products. However, they can also be employed in care cosmetics and decorative cosmetics.

The significant synergistic activity has been found for preparations according to the inventor comprising the mixture comprising or consisting of a) 1,2-decanediol and one or more compounds selected from constituent b) and additionally one or more compounds selected from constituent c), wherein the total amount of 1,2-decanediol in the preparation is in the range of from 0.1 to 2 wt.-% based on the weight of the preparation, including the preferred embodiments described herein, in which the content and the weight ration of constituents a) and b) are based on the total weight of the preparation as shown in table 1 above.

The synergistic active cosmetic or pharmaceutical, in particular dermatological preparations, which comprise a mixture comprising or consisting of a) 1,2-decanediol and b) one or more compounds selected from the group consisting of
a) an antimicrobial active amount of 1,2-decanediol of formula 1: 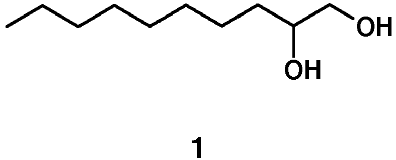 and
b) an antimicrobial active amount of one or more compounds selected from the group consisting of ethanol, propan-1-ol, propan-2-ol, chlorhexidine digluconate, chloroxylenol, triclosan, triclocarban, benzethonium chloride, methylbenzethonium chloride and benzalkonium chloride,
   wherein the preparation additionally comprises or consists of
c) an antimicrobial active amount of one or more compounds selected from the group consisting of mecetroniumetil sulfate, undecyleneamidopropyltrimonium methosulfate, (ethylendioxy)dimethanol, benzyl-C12-18-alkyldimethylammoniumchloride, didecyldimethylammonium chloride, N,N-didecyl-N-methyl-poly(oxethyl)ammonium propionate, N-(3-aminopropyl)-N-dodecylpropan-1,3-diamin, N-dodecylpropan-1,3-diamin, N-(3-aminopropyl)-N-dodecylpropan-1,3-diamin, clorofen, 2-biphenyl-2-ol, chlorocresol, hydrogen peroxide, acetic acid, peracetic acid, glutaral and formaldehyde,
wherein the total amount of 1,2-decanediol of formula 1 in the preparation is in the range of from 0.1 to 2 wt.-% based on the weight of the preparation,
including the preferred embodiments described herein, are preferably either in the form of a disinfecting liquid or solid soap, in the form of a disinfecting cleansing solution or in the form of a disinfecting emulsion.

Further conventional cosmetic and dermatological auxiliary substances and additives (including water) can be present in amounts of 5 - 99 wt.%, preferably 10 - 90 wt.%, based on the total weight of the preparation. Therefore, a preparation according to the invention, in particular in the form of disinfecting cleansing solution, including the preferred embodiments described herein, may regularly comprise additionally one or more compound of the following group of cleansing solution ingredients: water or aqueous (salt) solutions, further diols or polyols of low C number, preferably having 3 to 8 C atoms and ethers thereof, preferably propylene glycol (1,2-propanediol), glycerol, ethylene glycol, ethylene glycol monoethyl or monobutyl ether, propylene glycol monomethyl, monoethyl or monobutyl ether, diethylene glycol monomethyl or monoethyl ether and analogous products. Mixtures of the abovementioned solvents are used in particular. Water can be a further constituent. Further conventional cosmetic and dermatological auxiliary substances and additives (including water) can be present in amounts of 5 - 99 wt.%, preferably 10 - 90 wt.%, based on the total weight of the preparation.

In preferred embodiements conventional cosmetic and dermatological auxiliary substances and additives (including water) can be additionally present in amounts of 5 - 99 wt.%, preferably 10 - 90 wt.%, based on the total weight of the preparation. Therefore, a preparation according to the invention, in particular in the form of disinfecting liquid or solid soap, including the preferred embodiments of described herein, regularly comprises a surfactant system.

The surfactant system useful in this invention is comprised of amphoteric, nonionic, and cationic surfactants. Each of these surfactants are typically present in the antimicrobial system of this invention ranging from 0.1 to 15, preferably 0.1 to 8, most preferably 0.2 to 5% by weight.

Examples of suitable amphoteric surfactants include those related or derived from betaines such as amine betaines and amido betaines. Also useful amphoteric surfactants include glycinate and/or imidazole derivatives such as coco-imidazoline mono-carboxylate and/or dicarboxylate.

Nonionic surfactants are neutral molecules without any charge, and these compounds are very mild with poor foaming properties. Non-ionic compounds diminish surface tension and dissolve in water quite easily, but not in same way as common salt. They are equally soluble in oil, which is important in producing emulsions. In the presence of water, they do not form simple solutions, they form complexes known as hydrates. Applications for nonionics include solubilization and for cationics, conditioning. Examples: Alkyl phenol ethoxylates, fatty acid dialkanolamides, fatty acid monoalkanolamides, fatty acid ethoxylates, fatty alcohol ethoxylates, fatty amine ethoxylates, substituted phenol ethoxylates, vegetable oil ethoxylates, polyalkylglycosides, sucrose esters and glyceryl laurate.

Generally, preferred nonionic surfactants include condensation products of one or more alkylene oxide groups with an organic hydrophobic compound, such as an aliphatic or alkyl aromatic compound. Exemplary nonionic surfactants based upon polyethoxylated, polyproproxylated, or polyglyceroxylated alcohols, alkylphenols, or fatty acids.

Further specific examples of nonionic surfactants include, for example, alkyl phenoxypolyethoxy ethanols having alkyl groups from about 7 to 18 carbon atoms and from about 6 to about 60 oxyethylene units such as, for example, heptyl phenoxypolyethoxyethanols, ethylene oxide derivatives of long chained carboxylic acids such as lauric acid, myristic acid, palmitic acid, oleic acid, and the like, or mixtures of acids such as those found in tall oil containing from about 6 to 60 oxyethylene units; ethylene oxide condensates of long-chained alcohols such as octyl, decyl, lauryl, or cetyl alcohols containing from 6 to 60 oxyethylene units; ethylene oxide condensates of long-chain or branched chain amines such as dodecyl amine, hexadecyl amine, and octadecyl amine, containing from about 6 to 60 oxyetheylene units; and block copolymers of ethylene oxide sections combined with one of more hydrophobic propylene oxide sections.

Examples of cationic surfactants include, for example, lauryl pyridinium chloride, cetyldimethyl amine acetate, and alkyldimethylbenzylammonium chloride, in which the alkyl group has from 8 to 18 carbon atoms.

Other useful cationic surfactants include aliphatic fatty amines and their derivatives, homologues of aromatic amines having fatty chains--dodecylaniline, fatty amides derived from aliphatic diamines, fatty amides derived from disubstituted amines, quaternary ammonium compounds, amides derived from aminoalcohols and their quaternary ammonium derivatives, quaternary ammonium bases derived from fatty amides of disubstituted diamines, quaternary ammonium bases of the benzimidazolines, basic compounds of pyridinium and its derivatives, quaternary ammonium compound of betaine, dimethylphenylbenzyl ammonium chloride, urethanes or basic salts of ethylene diamine, polyethylene diamines and their quaternary ammonium compounds.

Further conventional cosmetic and dermatological auxiliary substances and additives (including water) can be present in amounts of 5 - 99 wt.%, preferably 10 - 90 wt.%, based on the total weight of the preparation.

A preparation according to the invention, in particular in the form of an O/W emulsion, including the preferred embodiments described herein, regularly comprises one or more of the following solvents: water or aqueous (salt) solutions, diols or polyols of low C number, preferably having 3 to 8 C atoms, and ethers thereof, preferably propylene glycol (1,2-propanediol), glycerol, ethylene glycol, ethylene glycol monoethyl or monobutyl ether, propylene glycol monomethyl, monoethyl or monobutyl ether, diethylene glycol monomethyl or monoethyl ether and analogous products. Mixtures of the above mentioned solvents are used in particular. Water can be a further constituent.

Further conventional cosmetic and dermatological auxiliary substances and additives (including water) can be present in amounts of 5 - 99 wt.%, preferably 10 - 90 wt.%, based on the total weight of the preparation.

A preparation according to the invention, including the preferred embodiments described herein, preferably in the form of an O/W emulsion, regularly comprises one or more of the following thickeners, which can advantageously be chosen from the group consisting of silicon dioxide, aluminium silicates, polysaccharides or derivatives thereof, e.g. hyaluronic acid, xanthan gum, hydroxypropylmethylcellulose, particularly advantageously from the group consisting of polyacrylates, preferably a polyacrylate from the group consisting of the so-called Carbopols, for example Carbopols of types 980, 981, 1382, 2984, 5984, in each case individually or in combination.

Preparations according to the invention in the form of an O/W emulsion, including the preferred embodiments described herein, advantageously comprise one or more emulsifiers.

O/W emulsifiers are advantageously chosen from the group consisting of polyethoxylated or polypropoxylated or polyethoxylated and polypropoxylated products, e.g.:
- the fatty alcohol ethoxylates
- the ethoxylated wool wax alcohols,
- the polyethylene glycol ethers of the general formula R-O-(-CH₂-CH₂-O-)ₙ-R',
- the fatty acid ethoxylates of the general formula R-COO-(-CH₂-CH₂-O-)ₙ-H,
- the etherified fatty acid ethoxylates of the general formula

   R-COO-(-CH₂-CH₂-O-)ₙ-R',
- the esterified fatty acid ethoxylates of the general formula

   R-COO-(-CH₂-CH₂-O-)ₙ-C(O)-R',
- the polyethylene glycol glycerol fatty acid esters
- the ethoxylated sorbitan esters
- the cholesterol ethoxylates
- the ethoxylated triglycerides
- the alkyl ether-carboxylic acids of the general formula
   R-COO-(-CH₂-CH₂-O-)ₙ-OOH, wherein n represents a number from 5 to 30,
- the polyoxyethylene sorbitol fatty acid esters
- the alkyl ether-sulfates of the general formula R-O-(-CH₂-CH₂-O-)ₙ-SO₃-H
- the fatty alcohol propoxylates of the general formula R-O-(-CH₂-CH(CH₃)-O-)ₙ-H
- the polypropylene glycol ethers of the general formula

   R-O-(-CH₂-CH(CH₃)-O-)ₙ-R'
- the propoxylated wool wax alcohols,
- the etherified fatty acid propoxylates R-COO-(-CH₂-CH(CH₃)-O-)ₙ-R'
- the esterified fatty acid propoxylates of the general formula

   R-COO-(-CH₂-CH(CH₃)-O-)ₙ-C(O)-R'
- the fatty acid propoxylates of the general formula

   R-COO-(-CH₂-CH(CH₃)-O-)ₙ-H,
- the polypropylene glycol glycerol fatty acid esters
- the propoxylated sorbitan esters
- the cholesterol propoxylates
- the propoxylated triglycerides
- the alkyl ether-carboxylic acids of the general formula

   R-O-(-CH₂-CH(CH₃)-O-)ₙ-CH₂-COOH,
- the alkyl ether-sulfates and the acids on which these sulfates are based of the general formula R-O-(-CH₂-CH(CH₃)-O-)ₙ-SO₃-H,
- the fatty alcohol ethoxylates/propoxylates of the general formula R-O-Xₙ-Yₘ-H
- the polypropylene glycol ethers of the general formula R-O-Xₙ-Yₘ-R'
- the etherified fatty acid propoxylates of the general formula R-COO-Xₙ-Yₘ-R'
- the fatty acid ethoxylates/propoxylates of the general formula R-COO-Xₙ-Yₘ-H.

According to the invention, the polyethoxylated or polypropoxylated or polyethoxylated and polypropoxylated O/W emulsifiers employed are particularly advantageously chosen from the group consisting of substances having HLB values of 11 - 18, very particularly advantageously having HLB values of 14.5 - 15.5, if the O/W emulsifiers contain saturated radicals R and R'. If the O/W emulsifiers contain unsaturated radicals R and/or R', or isoalkyl derivatives are present, the preferred HLB value of such emulsifiers can also be lower or higher.

It is of advantage to choose the fatty alcohol ethoxylates from the group consisting of ethoxylated stearyl alcohols, cetyl alcohols and cetylstearyl alcohols (cetearyl alcohols). The following are particularly preferred:
polyethylene glycol (n) stearyl ether (steareth-n), where n = 13-20,
polyethylene glycol (n) cetyl ether (ceteth-n), where n = 13-20,
polyethylene glycol (n) isocetyl ether (isoceteth-n), where n = 13-20,
polyethylene glycol (n) cetylstearyl ether (ceteareth-n), where n = 13-20,
polyethylene glycol (m) isostearyl ether (isosteareth-m), where m = 12-20
polyethylene glycol (k) oleyl ether (oleth-k), where k = 12-15
polyethylene glycol (12) lauryl ether (laureth-12),
polyethylene glycol (12) isolauryl ether (isolaureth-12).

It is furthermore advantageous to chose the fatty acid ethoxylates from the following group:
polyethylene glycol (n) stearate, where n = 20-25
polyethylene glycol (m) isostearate, where m = 12-25
polyethylene glycol (k) oleate, where k = 12-20

Sodium laureth-11 carboxylate can advantageously be used as an ethoxylated alkyl ether-carboxylic acid or salt thereof. Sodium laureth 1-4 sulfate can advantageously be used as an alkyl ether-sulfate. Polyethylene glycol (30) cholesteryl ether can advantageously be used as an ethoxylated cholesterol derivative. Polyethylene glycol (25) soyasterol has also proved suitable.

The polyethylene glycol (60) evening primrose glycerides can advantageously be used as ethoxylated triglycerides.

It is furthermore advantageous to chose the polyethylene glycol glycerol fatty acid esters from the group consisting of
polyethylene glycol (20-23) glyceryl-laurate polyethylene glycol (6) glyceryl-caprylate/caproate, polyethylene glycol (20) glyceryl-oleate, polyethylene glycol (20) glyceryl-isostearate, polyethylene glycol (18) glyceryl-oleate/cocoate.

It is likewise favourable to choose the sorbitan esters from the group consisting of polyethylene glycol (20) sorbitan monolaurate, polyethylene glycol (20) sorbitan monostearate, polyethylene glycol (20) sorbitan monoisostearate, polyethylene glycol (20) sorbitan monopalmitate and polyethylene glycol (20) sorbitan monooleate.

The (in particular topical) cosmetic or pharmaceutical, in particular dermatological preparations according to the invention, including the preferred embodiments described herein, can comprise cosmetic auxiliary substances and additives such as are conventionally used in such preparations, e.g. sunscreen agents, preservatives, further bactericides, further fungicides, further virucides, cooling active compounds, insect repellents (e.g. DEET, IR 3225, Dragorepel), plant extracts, antiinflammatory active compounds, substance which accelerate wound healing (e.g. chitin or chitosan and derivatives thereof), film-forming substances (e.g. polyvinylpyrrolidones or chitosan or derivatives thereof), the usual antioxidants, vitamins (e.g. vitamin C derivatives, tocopherols and derivatives, vitamin A and derivatives), skin care agents (e.g. cholesterol, ceramides, pseuodceramides), softening, moisturizing and/or humectant substances (in particular glycerol, urea or 1,2-alkanediols, such as 1,2-pentanediol, 1,2-hexanediol and/or 1,2-octanediol), saturated fatty acids, mono- or polyunsaturated fatty acids, alpha-hydroxy acids, polyhydroxy-fatty acids or derivatives thereof (e.g. linoleic acid, alpha-linolenic acid, gamma-linolenic acid or arachidonic acid and the particular natural or synthetic esters thereof), waxes or other conventional constituents of a cosmetic or dermatological preparation, such as alcohols, polyols, polymers, foam stabilizers, electrolytes, organic solvents, silicone derivatives, antidandruff active compounds (e.g. climbazole, ketoconazole, piroctonoleamine, zinc pyrithione), hair care agents, perfume, substances for preventing foaming, dyestuffs, pigments which have a colouring action, thickening agents, surface-active substances, surfactants, emulsifiers, plant parts and plant extracts (e.g. arnica, aloe, beard lichen, ivy, stinging nettle, ginseng, henna, camomile, marigold, rosemary, sage, blackberry, horsetail or thyme), royal jelly, propolis, proteins, protein hydrolysates, yeast extracts, hop and wheat extracts, peptides or thymus extracts.

The particular amounts of cosmetic or dermatological auxiliary substances and additives and of one or more odoriferous substances (perfumes) to be employed can be easily determined according to the nature of the particular product by simple trials by the person skilled in the art.

Preparations according to the invention, including the preferred embodiments described herein, optionally comprise one or more compounds having care properties, such as, for example, fatty alcohols having 6-30 C atoms. The fatty alcohols here can be saturated or unsaturated and linear or branched. Furthermore, these fatty alcohols can in some cases be a constituent of the oily phase (vii) if they correspond to the definition given there. Alcohols which can be employed are, for example, decanol, decenol, octanol, octenol, dodecanol, dodecenol, octadienol, decadienol, dodecadienol, oleyl alcohol, ricinoleyl alcohol, erucyl alcohol, stearyl alcohol, isostearyl alcohol, cetyl alcohol, lauryl alcohol, myristyl alcohol, arachidyl alcohol, caprylyl alcohol, capryl alcohol, linoleyl alcohol, linolenyl alcohol and behenyl alcohol, as well as Guerbet alcohols thereof, such as, for example, 2-octyl-1-dodecanol, it being possible for the list to be extended virtually as desired by further alcohols of related structural chemistry. The fatty alcohols preferably originate from natural fatty acids, being conventionally prepared from the corresponding esters of the fatty acids by reduction. Fatty alcohol fractions which are formed by reduction from naturally occurring fats and fatty oils, such as e.g. beef tallow, groundnut oil, colza oil, cottonseed oil, soya oil, sunflower oil, palm kernel oil, linseed oil, maize oil, castor oil, rape oil, sesame oil, cacao butter and coconut fat, can furthermore be employed.

Substances having care properties, specifically moisturising properties, refattening properties and barrier recovery properties which can be employed in an outstanding manner in the preparations according to the invention comprising a mixture comprising or consisting of a) 1,2-decanediol and one or more compounds selected from constituent b), including the preferred embodiments described herein, moreover include
- ceramides, where ceramides are understood as meaning N-acylsphingosins (fatty acid amides of sphingosin) or synthetic analogues of such lipids (so-called pseudo-ceramides), which significantly improve the water retention capacity of the stratum corneum,
- phospholipids, for example soya lecithin, egg lecithin and cephalins,
- fatty acids,
- phytosterols and phytosterol-containing fats or waxes,
- vaseline, paraffin oils and silicone oils; the latter include, inter alia, dialkyl- and alkylarylsiloxanes, such as dimethylpolysiloxane and methylphenylpolysiloxane, as well as alkoxylated and quaternized derivatives thereof.

Animal and/or plant protein hydrolysates can advantageously also be added to the preparations according to the invention, including the preferred embodiments described herein. Substances which are advantageous in this respect are, in particular, elastin, collagen, keratin, milk protein, soya protein, oat protein, pea protein, almond protein and wheat protein fractions or corresponding protein hydrolysates, and also condensation products thereof with fatty acids and quaternized protein hydrolysates, the use of plant protein hydrolysates being preferred.

The preparations according to the invention which comprise a mixture comprising or consisting of a) 1,2-decanediol and one or more compounds selected from constituent b), including the preferred embodiments for described herein, can additionally comprise one or more antioxidants, wherein antioxidants can be selected from generally available antioxidants, which are suitable or usual for cosmetic and/or dermatological uses.

The one of the antioxidants are advantageously chosen from the group consisting of amino acids (e.g. glycine, histidine, tyrosine, tryptophan) and derivatives thereof, imidazoles (e.g. urocanic acid) and derivatives thereof, peptides, such as D,L-carnosine, D-carnosine, L-carnosine and derivatives thereof (e.g. anserine), carotenoids, carotenes (e.g. alpha-carotene, beta-carotene, lycopene) and derivatives thereof, liponic acid and derivatives thereof (e.g. dihydroliponic acid), aurothioglucose, propyl-thiouracil and other thiols (e.g. thioredoxin, glutathione, cysteine, cystine, cystamine and glycosyl, N-acetyl, methyl, ethyl, propyl, amyl, butyl and lauryl, palmitoyl, oleyl, gamma-linoleyl, cholesteryl and glyceryl esters thereof) as well as salts thereof, dilauryl thiodipropionate, distearyl thiodipropionate, thiodipropionic acid and derivatives thereof (esters, ethers, peptides, lipids, nucleotides, nucleosides and salts) as well as sulfoximine compounds (e.g. buthionine sulfoximine, homocysteine sulfoximine, buthionine sulfones, penta-, hexa-, heptathionine sulfoximine) in very low tolerated dosages, furthermore (metal) chelators, e.g. alpha-hydroxy-fatty acids, palmitic acid, phytic acid, lactoferrin, alpha-hydroxy acids (e.g. citric acid, lactic acid, malic acid), humic acid, bile acid, bile extracts, bilirubin, biliverdin, EDTA, EGTA and derivatives thereof, unsaturated fatty acids and derivatives thereof (e.g. gamma-linolenic acid, linoleic acid, oleic acid), folic acid and derivatives thereof, ubiquinone and ubiquinol and derivatives thereof, vitamin C derivatives (e.g. ascorbyl palmitate, Mg ascorbyl phosphate, ascorbyl acetate), tocopherols and derivatives thereof (e.g. vitamin E, vitamin E acetate), vitamin A and derivatives thereof (vitamin A palmitate) as well as coniferylbenzoate of benzoin resin, rutic acid and derivatives thereof, ferulic acid and derivatives thereof, butylhydroxytoluene, butylhydroxyanisole, nordihydroguaiac resin acid, nordihydroguaiaretic acid, trihydroxybutyrophenone, uric acid and derivatives thereof, mannose and derivatives thereof, zinc and derivatives thereof (e.g. ZnO, ZnSO₄), selenium and derivatives thereof (e.g. selenium methionine), stilbenes and derivatives thereof (e.g. stilbene oxide, trans-stilbene oxide) and derivatives (salts, esters, ethers, sugars, nucleotides, nucleosides, peptides and lipids) of these active compounds mentioned.

The preparations according to the invention, which comprise a mixture comprising or consisting of a) 1,2-decanediol and one or more compounds selected from constituent b), including the preferred embodiments described herein, can advantageously additionally comprise vitamins and vitamin precursors, it being possible for all the vitamins and vitamin precursors which are suitable or usual for cosmetic and/or dermatological uses to be used. There are worth mentioning here, in particular, vitamins and vitamin precursors, such as tocopherols, vitamin A, niacin acid and niacinamide, further vitamins of the B complex, in particular biotin, and vitamin C and panthenol and derivatives thereof, in particular the esters and ethers of panthenol and cationically derivatized panthenols, such as e.g. panthenol triacetate, panthenol monoethyl ether and the monoacetate thereof and cationic panthenol derivatives.

The preparations according to the invention, which comprise a mixture comprising or consisting of a) 1,2-decanediol and one or more compounds selected from constituents b), including the preferred embodiments described herein, can additionally comprise one or more antiinflammatory and/or redness- and/or itching-alleviating active compounds. All the antiinflammatory or redness- and/or itching-alleviating active compounds which are suitable or usual for cosmetic and/or pharmaceutical, in particular dermatological uses can be used here. Antiinflammatory and redness- and/or itching-alleviating active compounds which are advantageously employed are steroidal antiinflammatory substances of the corticosteroid type, such as e.g. hydrocortisone, dexamethasone, dexamethasone phosphate, methylprednisolone or cortisone, it being possible for the list to be extended by addition of further steroidal antiinflammatories. Non-steroidal antiinflammatories can also be employed. There are to be mentioned here by way of example oxicams, such as piroxicam or tenoxicam; salicylates, such as aspirin, disalcid, solprin or fendosal; acetic acid derivatives, such as diclofenac, fenclofenac, indomethacin, sulindac, tolmetin or clindanac; fenamates, such as mefenamic, meclofenamic, flufenamic or niflumic; propionic acid derivatives, such as ibuprofen, naproxen, benoxaprofen or pyrazoles, such as phenylbutazone, oxyphenylbutazone, febrazone or azapropazone. Alternatively, natural antiinflammatory or redness- and/or itching-alleviating substances can be employed. Plant extracts, specific highly active plant extract fractions and highly pure active substances isolated from plant extracts can be employed. Extracts, fractions and active substances from camomile, aloe vera, Commiphora species, Rubia species, willow, rose-bay willow herb, oats as well as pure substances, such as, inter alia, bisabolol, apigenin 7-glucoside, boswellic acid, phytosterols, glycyrrhizic acid, glabridin or licochalcone A, are particularly preferred. The preparations comprising diphenylmethane derivatives of the formula 1 can also comprise mixtures of two or more antiinflammatory active compounds.

Bisabolol, boswellic acid, as well as extracts and isolated highly pure active compounds from oats and Echinacea are particularly preferred for use in the context of the invention, and alpha-bisabolol and extracts and isolated highly pure active compounds from oats are especially preferred.

The amount of antiirritants (one or more compounds) in the preparations is preferably 0.0001 to 20 wt.%, particularly preferably 0.0001 to 10 wt.%, in particular 0.001 to 5 wt.%, based on the total weight of the preparation.

The preparations according to the invention, which comprise a mixture comprising or consisting of a) 1,2-decanediol and one or more compounds selected from constituent b), including the preferred embodiments described herein, can advantageously additionally comprise moisture retention regulators. The following substances, e.g. are used as moisture retention regulators (moisturizers): sodium lactate, urea, alcohols, sorbitol, glycerol, propylene glycol, collagen, elastin or hyaluronic acid, diacyl adipates, petrolatum, ectoin, urocanic acid, lecithin, pantheol, phytantriol, lycopene, algae extract, ceramides, cholesterol, glycolipids, chitosan, chondroitin sulfate, polyamino acids lanolin, lanolin esters, amino acids, alpha-hydroxy acids (e.g. citric acid, lactic acid, malic acid) and derivatives thereof, sugars (e.g. inositol), alpha-hydroxy-fatty acids, phytosterols, triterpene acids, such as betulinic acid or ursolic acid, algae extracts.

The preparations according to the invention which comprise a mixture comprising or consisting of a) 1,2-decanediol and one or more compounds selected from constituent b), including the preferred embodiments described herein, can advantageously additionally comprise mono-, di- and oligosaccharides, such as, for example, glucose, galactose, fructose, mannose, laevulose and lactose.

The preparations according to the invention which comprise a mixture comprising or consisting of a) 1,2-decanediol and one or more compounds selected from constituent b), including the preferred embodiments described herein, can advantageously additionally comprise plant extracts, which are conventionally prepared by extraction of the whole plant, but also in individual cases exclusively from blossom and/or leaves, wood, bark or roots of the plant. In respect of the plant extracts which can be used, reference is made in particular to the extracts which are listed in the table starting on page 44 of the 3rd edition of the Leitfaden zur Inhaltsstoffdeklaration kosmetischer Mittel [Manual of Declaration of the Constituents of Cosmetic Compositions], published by Industrieverband Körperpflegemittel und Waschmittel e.V. (IKW), Frankfurt. Extracts which are advantageous in particular are those from aloe, witch hazel, algae, oak bark, rose-bay willow-herb, stinging nettle, dead nettle, hops, camomile, yarrow, arnica, calendula, burdock root, horsetail, hawthorn, linden blossom, almond, pine needle, horse chestnut, sandalwood, juniper, coconut, mango, apricot, orange, lemon, lime, grapefruit, apple, green tea, grapefruit pip, wheat, oats, barley, sage, thyme, wild thyme, rosemary, birch, mallow, lady's smock, willow bark, restharrow, coltsfoot, hibiscus, ginseng and ginger root. In this context, the extracts from aloe vera, camomile, algae, rosemary, calendula, ginseng, cucumber, sage, stinging nettle, linden blossom, arnica and witch hazel are particularly preferred. Mixtures of two or more plant extracts can also be employed. Extraction agents which can be used for the preparation of the plant extracts mentioned are, inter alia, water, alcohols and mixtures thereof. In this context, among the alcohols lower alcohols, such as ethanol, propan-1-ol and propan-2-ol, but also polyhydric alcohols, such as ethylene glycol, propylene glycol and butylene glycol, are preferred, and in particular both as the sole extraction agent and in mixtures with water. The plant extracts can be employed both in the pure and in the diluted form.

Preparations according to the invention, including the preferred embodiments described herein, can in numerous cases advantageously comprise the one or more compounds of following preservatives. Such preservatives even can enhance the disinfecting properties of the inventive preparations comprising a mixture comprising or consisting of a) 1,2-decanediol and one or more compounds selected from constituent b). Preservatives which are preferably chosen here are those such as benzoic acid, its esters and salts, propionic acid and its salts, salicylic acid and its salts, 2,4-hexadienoic acid (sorbic acid) and its salts, formaldehyde and paraformaldehyde, 2-hydroxybiphenyl ether and its salts, 2-zinc-sulfidopyridine N-oxide, inorganic sulfites and bisulfites, sodium iodate, chlorobutanolum, 4-ethylmercury(II)-5-amino-1,3-bis(2-hydroxybenzoic acid), its salts and esters, dehydracetic acid, formic acid, 1,6-bis(4-amidino-2-bromophenoxy)-n-hexane and its salts, the sodium salt of ethylmercury(II)-thiosalicylic acid, phenylmercury and its salts, 10-undecylenic acid and its salts, 5-amino-1,3-bis(2-ethylhexyl)-5-methyl-hexahydropyrimidine, 5-bromo-5-nitro-1,3-dioxane, 2-bromo-2-nitro-1,3-propanediol, 2,4-dichlorobenzyl alcohol, 4-chloro-m-cresol, 4-chloro-3,5-dimethylphenol, 1,1'-methylene-bis(3-(1-hydroxymethyl-2,4-dioximidazolidin-5-yl)urea), poly-(hexamethylene diguanide) hydrochloride, 2-phenoxyethanol, hexamethylenetetramine, 1-(3-chloroallyl)-3,5,7-triaza-1-azonia-adamantane chloride, 1-(4-chlorophenoxy)-1-(1H-imidazol-1-yl)-3,3-dimethyl-2-butanone, 1,3-bis-(hydroxy-methyl)-5,5-dimethyl-2,4-imidazolidinedione, benzyl alcohol, Octopirox, 1,2-dibromo-2,4-dicyanobutane, 2,2'-methylene-bis(6-bromo-4-chloro-phenol), bromochlorophene, mixture of 5-chloro-2-methyl-3(2H)-isothiazolinone and 2-methyl-3(2H)-isothiazolinone with magnesium chloride and magnesium nitrate, 2-benzyl-4-chlorophenol, 2-chloroacetamide, 1-phenoxy-propan-2-ol, N-alkyl(C₁₂-C₂₂)trimethyl-ammonium bromide and chloride, 4,4-dimethyl-1,3-oxazolidine, N-hydroxymethyl-N-(1,3-di(hydroxymethyl)-2,5-dioxoimidazolidin-4-yl)-N'-hydroxy-methylurea, 1,6-bis(4-amidino-phenoxy)-n-hexane and its salts, glutaraldehyde, 5-ethyl-1-aza-3,7-dioxabicyclo(3.3.0)octane, 3-(4-chlorophenoxy)-1,2-propanediol, hyamines, alkyl-(C₈-C₁₈)-dimethyl-benzyl-ammonium chloride, alkyl-(C₈-C₁₈)-dimethyl-benzylammonium bromide, alkyl-(C₈-C₁₈)-dimethyl-benzyl-ammonium saccharinate, benzyl hemiformal, 3-iodo-2-propynyl butylcarbamate or sodium hydroxymethyl-aminoacetate.

In various cases it may also be advantageous to employ further substances which are chiefly employed for inhibition of the growth of undesirable microorganisms on or in animal organisms in the preparations according to the invention comprising a mixture comprising or consisting of a) 1,2-decanediol and one or more compounds selected from constituent b), including the preferred embodiments described herein. In this respect, in addition to conventional preservatives, further active compounds which are worth mentioning, in addition to the large group of conventional antibiotics, are, in particular, the products relevant for cosmetics, such as climbazole, octoxyglycerol, Octopirox (1-hydroxy-4-methyl-6-(2,4,4-trimethylpentyl)-2(1H)-pyridone, 2-aminoethanol), chitosan, farnesol, glycerol monolaurate or combinations of the substances mentioned, which are employed, inter alia, against underarm odour, foot odour or dandruff formation. Also these compounds may additionally enhance the disinfecting properties of the inventive preparation comprising mixture comprising or consisting of a) 1,2-decanediol and one or more compounds selected from constituent b).

Further to this the preparations according to the invention comprising a mixture comprising or consisting of a) 1,2-decanediol and one or more compounds selected from constituent, including the preferred embodiments described herein, can further comprise one or more cooling agents.

Individual cooling active compounds which are preferred for use in the context of the present invention are listed below. The person skilled in the art can supplement the following list with a large number of further cooling active compounds; the cooling active compounds listed can also be employed in combination with one another: l-menthol, d-menthol, racemic menthol, menthone glycerol acetal (trade name: Frescolat®MGA), menthyl lactate (trade name: Frescolat®ML, menthyl lactate is preferably l-menthyl lactate, in particular l-menthyl l-lactate), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxyethyl menthyl carbonate, 2-hydroxypropyl menthyl carbonate, N-acetylglycine menthyl ester, isopulegol, menthyl hydroxycarboxylic acid esters (e.g. menthyl 3-hydroxybutyrate), monomenthyl succinate, 2-mercaptocyclodecanone, menthyl 2-pyrrolidin-5-onecarboxylate, 2,3-dihydroxy-p-menthane, 3,3,5-trimethylcyclohexanone glycerol ketal, 3-menthyl 3,6-di- and -trioxaalkanoates, 3-menthyl methoxyacetate, icilin.

Preferred cooling active compounds are: I-menthol, d-menthol, racemic menthol, menthone glycerol acetal (trade name: Frescolat®MGA), menthyl lactate (preferably l-menthyl lactate, in particular l-menthyl l-lactate, trade name: Frescolat®ML), substituted menthyl-3-carboxylic acid amides (e.g. menthyl-3-carboxylic acid N-ethylamide), 2-isopropyl-N-2,3-trimethylbutanamide, substituted cyclohexanecarboxylic acid amides, 3-menthoxypropane-1,2-diol, 2-hydroxyethyl menthyl carbonate, 2-hydroxypropyl menthyl carbonate, isopulegol.

Particularly preferred cooling active compounds are: I-menthol, racemic menthol, menthone glycerol acetal (trade name: Frescolat®MGA), menthyl lactate (preferably l-menthyl lactate, in particular l-menthyl l-lactate, trade name: Frescolat®ML), 3-menthoxypropane-1,2-diol, 2-hydroxyethyl menthyl carbonate, 2-hydroxypropyl menthyl carbonate.

Very particularly preferred cooling active compounds are: I-menthol, menthone glycerol acetal (trade name: Frescolat®MGA), menthyl lactate (preferably l-menthyl lactate, in particular l-menthyl l-lactate, trade name: Frescolat®ML).

The use concentration of the cooling active compounds to be employed is, depending on the substance, preferably in the concentration range of from 0.01 to 20 wt.% and preferably in the concentration range of from 0.1 to 5 wt.%, based on the total weight of the finished (ready-to-use) cosmetic or pharmaceutical preparation.

The preparations according to the invention which comprise a mixture comprising or consisting of a) 1,2-decanediol and one or more compounds selected from constituent b), including the preferred embodiments described herein, can additionally comprise anionic, cationic, nonionic and/or amphoteric surfactants, especially if crystalline or microcrystalline solids, for example inorganic micropigments, are to be incorporated into the preparations. Surfactants are amphiphilic substances which can dissolve organic, nonpolar substances in water. According to the invention, surfactants therefore do not belong to the oily phase. In this context, the hydrophilic contents of a surfactant molecule are usually polar functional groups, for example -COO⁻, -OSO₃²⁻, -SO₃⁻, while the hydrophobic parts as a rule are nonpolar hydrocarbon radicals. Surfactants are in general classified according to the nature and charge of the hydrophilic molecular moiety. A distinction can be made between four groups here:
- anionic surfactants,
- cationic surfactants,
- amphoteric surfactants and
- nonionic surfactants.

Anionic surfactants as a rule contain carboxylate, sulfate or sulfonate groups as functional groups. In aqueous solution, they form negatively charged organic ions in an acid or neutral medium. Cationic surfactants are almost exclusively characterized by the presence of a quaternary ammonium group. In aqueous solution, they form positively charged organic ions in an acid or neutral medium. Amphoteric surfactants contain both anionic and cationic groups and accordingly behave like anionic or cationic surfactants in aqueous solution, depending on the pH. In a strongly acid medium they have a positive charge, and in an alkaline medium a negative charge. On the other hand, they are zwitter-ionic in the neutral pH range. Polyether chains are typical of nonionic surfactants. Nonionic surfactants do not form ions in an aqueous medium.

### A. Anionic surfactants

Anionic surfactants which are advantageously to be used are acylamino acids (and salts thereof), such as
- acyl glutamates, for example sodium acyl glutamate, di-TEA-palmitoyl aspartate and sodium caprylic/capric glutamate,
- acyl peptides, for example palmitoyl hydrolysed milk protein, sodium cocoyl hydrolysed soya protein and sodium/potassium cocoyl hydrolysed collagen,
- sarcosinates, for example myristoyl sarcosine, TEA-lauroyl sarcosinate, sodium lauroyl sarcosinate and sodium cocoyl sarcosinate,
- taurates, for example sodium lauroyl taurate and sodium methylcocoyl taurate,
- acyl lactylates, lauroyl lactylate, caproyl lactylate
- alaninates
   carboxylic acids and derivatives, such as
- for example, lauric acid, aluminium stearate, magnesium alkanolate and zinc undecylenate,
- ester-carboxylic acids, for example calcium stearoyl lactylate, laureth-6 citrate and sodium PEG-4 lauramide carboxylate,
- ether-carboxylic acids, for example sodium laureth-13 carboxylate and sodium PEG-6 cocamide carboxylate,
   phosphoric acid esters and salts, such as, for example, DEA-oleth-10 phosphate and dilaureth-4 phosphate,
   sulfonic acids and salts, such as
- acyl isethionates, e.g. sodium/ammonium cocoyl isethionate,
- alkylarylsulfonates,
- alkylsulfonates, for example sodium coco-monoglyceride sulfate, sodium C₁₂₋₁₄ olefin-sulfonate, sodium lauryl sulfoacetate and magnesium PEG-3 cocamide sulfate,
- sulfosuccinates, for example dioctyl sodium sulfosuccinate, disodium laureth-sulfosuccinate, disodium laurylsulfosuccinate and disodium undecylenamido-MEA-sulfosuccinate
   and
   sulfuric acid esters, such as
- alkyl ether-sulfate, for example sodium, ammonium, magnesium, MIPA, TIPA laureth sulfate, sodium myreth sulfate and sodium C12-13 pareth sulfate,
- alkyl sulfates, for example sodium, ammonium and TEA lauryl sulfate.

### B. Cationic surfactants

Cationic surfactants which are advantageously to be used are
- alkylamines,
- alkylimidazoles,
- ethoxylated amines and
- quaternary surfactants,
   RNH₂CH₂CH₂COO⁻ (at pH=7)
   RNHCH₂CH₂COO⁻ B⁺ (at pH=12) B⁺ = any desired cation, e.g. Na⁺
- ester quats.

Quaternary surfactants contain at least one N atom which is covalently bonded to 4 alkyl or aryl groups. This leads to a positive charge, independently of the pH. Alkylbetaine, alkylamidopropylbetaine and alkylamidopropylhydroxysulfaine are advantageous. The cationic surfactants used can furthermore preferably be chosen from the group consisting of quaternary ammonium compounds, in particular benzyltrialkyl-ammonium chlorides or bromides, such as, for example, benzyldimethylstearyl-ammonium chloride, furthermore alkyltrialkylammonium salts, for example cetyltrimethylammonium chloride or bromide, alkyldimethylhydroxy-ethylammonium chlorides or bromides, dialkyldimethylammonium chlorides or bromides, alkylamide-ethyltrimethyl-ammonium ether-sulfates, alkylpyridinium salts, for example lauryl- or cetylpyrimidinium chloride, imidazoline derivatives and compounds having a cationic character, such as amine oxides, for example alkyldimethylamine oxides or alkylaminoethyldimethylamine oxides. Cetyltrimethyl-ammonium salts in particular are advantageously to be used.

### C. Amphoteric surfactants

Amphoteric surfactants which are advantageously to be used are
a) acyl-/dialkylethylenediamine, for example sodium acylamphoacetate, disodium acylamphodipropionate, disodium alkylamphodiacetate, sodium acyl-amphohydroxy-propylsulfonate, disodium acylamphodiacetate and sodium acylamphopropionate,
b) N-alkylamino acids, for example aminopropyl alkylglutamide, alkylaminopropionic acid, sodium alkylimidodipropionate and lauroamphocarboxyglycinate.

### D. Nonionic surfactants

Nonionic surfactants which are advantageously to be used are
- alcohols,
- alkanolamides, such as cocamides MEA/DEA/MIPA,
- amine oxides, such as cocoamidopropylamine oxide,
- esters which are formed by esterification of carboxylic acids with ethylene oxide, glycerol, sorbitan or other alcohols,
- ethers, for example ethoxylated/propoxylated alcohols, ethoxylated/propoxylated esters, ethoxylated/propoxylated glycerol esters, ethoxylated/propoxylated cholesterols, ethoxylated/propoxylated triglyceride esters, ethoxylated/propoxylated lanolin, ethoxylated/propoxylated polysiloxanes, propoxylated POE ethers and alkyl polyglycosides, such as lauryl glucoside, decyl glycoside and coco-glycoside.
- sucrose esters, sucrose ethers
- polyglycerol esters, diglycerol esters, monoglycerol esters
- methylglucose esters, esters of hydroxy acids

The use of a combination of anionic and/or amphoteric surfactants with one or more nonionic surfactants is furthermore advantageous.

In this context, the surface-active substance(s) can be present in a preparation according to the invention in an amount in the range of from 0.5 to 98 wt.%, based on the total weight of the preparation.

Preferred embodiments and further preferred aspects of the present invention emerge from the attached patent claims and the following examples. Unless stated otherwise, all the data relate to the weight. The subject matter of the present invention, however, is not limited on the following examples.

Example 1: *In vitro* experiments on the synergistic antimicrobial efficacy of a preparation according to the invention comprising a) 1,2-decanediol and at least one compound selected from the group in constituent b).

The finding that mixtures of 1,2-decanediol in an antimicrobial effective amount together with an antimicrobial effective amount of one or more compounds selected from the group of constituent b) of the preparation or the mixture according to the present invention improves antimicrobial activity in a synergistic way is based on microbial tests, e.g. according to European DIN EN 12054, approved for the study of the efficacy disinfectant properties of liquid and solid soaps or disinfectant cleansing solutions for hygiene and surgery applications.

### Results:

The studies show that mixtures comprising an antimicrobial effective amount of 1,2-decanediol and an antimicrobial effective amount of one or more compounds selected from the group in constituent b) consisting of one or more compounds selected from the group consisting of ethanol (CARN 64-17-5; INCI name: Alcohol), propan-1-ol (CARN 71-23-8; INCI name: Propyl Alcohol), propan-2-ol (CARN 67-63-0; INCI name: Isopropyl Alcohol), chlorhexidine digluconate (CARN 18472-51-0; INCI name: Chlorhexidine Digluconate), chloroxylenol (CARN 88-04-0; 1321-23-9; INCI name: Chloroxylenol), triclosan (CARN 3380-34-5; INCI name: Triclosan), triclocarban (CARN 101-20-2; 1322-40-3; INCI name: Triclocarban), benzethonium chloride (CARN 121-54-0; INCI name: Benzethonium Chloride), methylbenzethonium chloride (CARN 25155-18-4; INCI name: Methylbenzethonium Chloride) and benzalkonium chloride (CARN 8001-54-5, 61789-71-7, 68391-01-5, 68424-85-1, 85409-22-9, INCI name: Benzalkonium Chloride) in the specific weight ratios listed in table 2 had a synergistic antimicrobial activity. The synergistic activity was confirmed via calculation of synergy indices with Kulls' equation according to the method described in F.C. Kull et al. (Applied Microbiology 9, p. 538, 1961) and D.C.Steinberg (Cosmetics & Toiletries 115 (11), p. 59, 2000), respectively.

**Table 2: Synergistically active ratios of a) 1,2-decanediol and another antimicrobial agent selected from constituent b) according to the preparation of the invention in the quantitative suspension test according to DIN EN12054.**

| **Antimicrobial Agents Ratio a) 1,2-decanediol : b) further antimicrobial agent** | | | | | | |
|---|---|---|---|---|---|---|
| ethanol | 1 : 500 | 1 : 200 | 1 : 100 | 1 : 150 | 1 : 75 | 1 : 250 |
| propyl alcohol | 1 : 200 | 1 : 100 | 1 : 500 | 1 : 250 | 1 : 150 | 1 : 75 |
| isopropyl alcohol | 1 : 200 | 1 : 100 | 1 : 500 | 1 : 250 | 1 : 150 | 1 : 75 |
| triclosan | 10 : 1 | 1 : 5 | 5 : 1 | 2.5 : 1 | 1 : 5 | 7.5 : 1 |
| triclocarban | 1 : 10 | 10 : 1 | 2.5 : 1 | 1 : 7.5 | 3 : 1 | 1:4 |
| chlorohexidine digluconate | 20 : 1 | 1 : 10 | 5 : 1 | 1 : 8 | 3 : 1 | 1 :15 |
| chloroxylenol | 5 : 1 | 1 : 1 | 2 : 1 | 1 : 20 | 1 : 10 | 1 : 2 |
| benzethonium chloride | 10 : 1 | 1 : 2 | 15 : 1 | 20 : 1 | 1 : 1 | 5 : 1 |
| methylbenzethonium chloride | 1 : 5 | 25 : 1 | 1 : 1 | 50 : 1 | 2 : 1 | 10 : 1 |
| benzalkonium chloride | 1 : 50 | 5 : 1 | 1 : 25 | 10 : 1 | 2 : 3 | 1 : 10 |

Example 2: Examples of preparations according to the invention comprising synergistic active mixtures of a) 1,2-decanediol and one or more antimicrobial agents from the group of constituent b).

Cosmetic and pharmaceutical preparations according to the invention which show particularly enhanced synergistic antimicrobial effects are further described in table 3.

A further improvement of the antimicrobial efficacy is effected when the combination is further combined with one or more additional antimicrobial agents not listed in the group of constituent b). Preferred embodiments of the present invention emerge from the following table and the attached patent claims.

**Table 3: Cosmetic and pharmaceutical preparations comprising a) an antimicrobial effective amount of 1,2-decanediol and one or more antimicrobial active compounds selected from the group of constituent b).**

| | | | | | | | | | | | |
|---|---|---|---|---|---|---|---|---|---|---|---|
| Preparation 1: Disinfectant surface cleaner (kitchen) | | | | | | | | | | | |
| Preparation 2: Disinfectant surface cleaner (bathroom) | | | | | | | | | | | |
| Preparation 3: Surface disinfectant solution | | | | | | | | | | | |
| Preparation 4: Hand disinfectant solution | | | | | | | | | | | |
| Preparation 5: Hand disinfectant solution for sensitive skin | | | | | | | | | | | |
| Preparation 6: Disinfectant soap bar | | | | | | | | | | | |
| Preparation 7: Disinfectant liquid soap | | | | | | | | | | | |
| Preparation 8: Solution for (medical) instrument disinfection | | | | | | | | | | | |
| Preparation 9: Hand cream with disinfectant properties | | | | | | | | | | | |
| Preparation 10: Disinfectant all purpose cleaner | | | | | | | | | | | |

| **Ingredient** | **INCI Name/Chemical Name** | | | | | | | | | | |
|---|---|---|---|---|---|---|---|---|---|---|---|
| | | **1** | **2** | **3** | **4** | **5** | **6** | **7** | **8** | **9** | **10** |
| **1,2 Decanediol** | **Decylene Glycol** | 0,5 | 0,5 | 1,0 | 0,5 | 1.0 | 2,0 | 2,0 | 0,5 | 2,0 | 0,5 |
| **Benzalkonium Chloride** | **Benzalkonium Chloride** | | 5,0 | | | | | | | | |
| **Benzethonium Chloride** | **Benzethonium Chloride** | | | 0,5 | | | | | | | |
| **Chlorohexidine Digluconate** | **Chlorohexidine Digluconate** | 1,0 | | | | | | | | | |
| **Chloroxylenol** | **Chloroxylenol** | | | | | | | | 2 | | |
| **Ethanol 96%** | **Alcohol** | | | | | 95,0 | | | | | |
| **Irgasan DP 300** | **Triclosan** | | | | | | | | | 0,3 | |
| **Methylbenzethonium Chloride** | **Methylbenzethonium Chloride** | | | | 0,2 | | | 1,0 | | | |
| **Preventol SB** | **Triclocarban** | | | | | | 0,5 | | | | |
| **Propan-1-ol** | **Propyl Alcohol** | | | | 75,0 | | | | | | 60 |
| **Propan-2-ol** | **Isopropyl Alcohol** | 10,0 | 15,0 | 75,0 | 4,0 | | | | | | |
| Bardac 22 | Didecyldimethyl-ammoniummchloride | 0,2 | 0,2 | | | | | | | | |
| Benzyl-C12-18 alkyldimethylammonium-chloride | Benzyl-C12-18 alkyldimethylammonium-chloride | | | | | | | | 1,0 | | |
| (-) alpha Bisabolol | Bisabolol | | | | 0,1 | 0,1 | | | | | |
| Abil 350 | Dimethicone | | | | | | | | | 0,3 | |
| Citric Acid 10% sol. | Citric Acid | | | | | | | 0,8 | | 0,1 | |
| Citric Acid Monohydrate crystals | Citric Acid | | 1,2 | | | | | | | | 0,4 |
| Comperlan KD | Cocamide DEA | | | | | | | 2,0 | | | |
| Didecyldimethyl-ammoniumchloride | Didecyldimethylammoniumchlorid | | | | | | | | 1,0 | | |
| Dissolvine A-40 | Nitrilotriacetic acid, Trisodium salt | | | | | | | | | | 0,5 |
| Dracorin 100 s.e. P | Glyceryl Stearate, PEG-100 Stearate | | | | | | | | | 6,0 | |
| Dragocid Liquid | Phenoxyethanol, Methylparaben, Ethylparaben, Butylparaben, Propylparaben, Isobutylparaben | | | | | | | 0,8 | | 0,5 | |
| Dragoderm | Glycerin, Triticum Vulgare (Wheat) Gluten, Water (Aqua) | | | | | | | | | 1,0 | |
| Dragosantol 100 | Bisabolol | | | | | | 0,1 | | | | |
| Dragoxat 89 | Ethylhexyl Isononanoate | | | | | | | | | 3,0 | |
| Elfan NS 252 S | Sodium C12-15 Pareth Sulfate | | | | | | | 40,0 | | | |
| Ethylendioyx)dimethanol | (Ethylendioyx)dimethanol | | | | | | | | 15,3 | | |
| Eumulgin B2 | Ceteareth-20 | | | | | | | | | 0,5 | |
| Farnesol | Farnesol | | | | 0,2 | 0,2 | | | | | |
| Fragrance | Fragrance | 0,1 | 0,2 | 0,1 | 0,1 | 0,1 | 0,2 | 0,5 | 0,1 | 0,3 | |
| Glutaral | Glutaral | | | | | | | | 7,5 | | |
| Glycerin 99,5 P | Glycerin | | | | 1,0 | | | | | | |
| Hydrogen Peroxide 30% | Hydrogen Peroxide | | | | | | | | | | 10,0 |
| Hydrolite-5 | Pentylene Glycol | | | | | | | | | 3,0 | |
| Hydroviton-24 | Water, Pentylene Glycol, Glycerin, Lactic Acid, Sodium Lactate, Serine, Urea, Sorbitol, Sodium Chloride, Allantoin | | | | 0,5 | 0,5 | | | | | |
| Imbentin AG/124/060 | Fatty alcohol ethoxylate (C12-14, 6EO) | 0,1 | 0,1 | 0,2 | | | | | | | 1,0 |
| Imbentin T/080 | Oxoalcohol C13, 8 Mol EO | | | | | | | | | | 3,0 |
| Iso Adipat | Diisopropyl Adipate | | | | 0,5 | | | | | | |
| Isodragol | Triisononanoin | | | | | 0,5 | | | | | |
| Kelzan T | Xanthan Gum | | | | | | | | | | 0,4 |
| K-Oleat 20% VZ NP | Potassium Oleate | | | | | | | | | | 1,0 |
| Lanette 16 | Cetyl Alcohol | | | | | | | | | 2,0 | |
| Mineral Oil | | | | | | | | | | 5,0 | |
| PCL Liquid 100 | Cetearyl Ethylhexanoate | | | | | | | | | 1,0 | |
| Soap Bar | Sodium Tallowate, Sodium Cocoate, Sodium Palm Kernelate, Aqua, Glycerin, Sodium Chloride, Tetrasodium Etidronate | | | | | | 95,0 | | | | |
| Sodium Chloride | Sodium Chloride | | | | | | | 1,0 | | | |
| Sodium Metasilicate Pentahydrate | Sodium Metasilicate Pentahydrate | 0,05 | | 0,05 | | | | | | | |
| Solubilizer | PEG-40 Hydrogenated Castor Oil, Trideceth-9, Propylene Glycol, Water (Aqua) | | | | | | | 1,0 | | | 1,5 |
| SymCalmin | Butylene Glycol, Pentylene Glycol, Hydroxyphenyl Propamidobenzoic Acid | | | | | | | | | 1,0 | |
| SymDeo MPP | Dimethyl Phenyl 2-Butanol | | | | | | | | | | 0,5 |
| Symdiol 68 | 1,2-Hexanediol, Caprylyl Glycol | | | | 0,5 | 0,5 | | | | | |
| SymMoillent W/S | Trideceth-9, PEG-5 Isononanoate, Water | | | | 1,0 | 2,0 | | 1,5 | | | |
| SymRelief | Bisabolol, Zingiber Officinale (Ginger) Root Extract | | | | | 0,1 | | | | | |
| SymRepair | Hexyldecanol, Bisabolol, Cetylhydroxyproline Palmitamide, Stearic Acid, Brassica Campestris (Rapeseed Sterols) | | | | 1,0 | 0,5 | | | | | |
| Tego Betain L7 | Cocamidopropyl Betaine | | | | | | | 5,0 | | | |
| Trisodium Citrate Dihydrate | Trisodium Citrate | | 0,2 | | | | | | | | |
| Water, deionized | Water (Aqua) | Ad100 | Ad100 | Ad100 | Ad100 | Ad100 | Ad100 | Ad100 | Ad100 | Ad100 | Ad100 |

## Claims

1. Cosmetic, pharmaceutical and/or household product preparation, comprising a mixture comprising or consisting of
a) an antimicrobial active amount of 1,2-decanediol of formula 1: 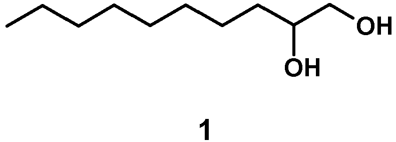 and
b) an antimicrobial active amount of one or more compounds selected from the group consisting of ethanol, propan-1-ol, propan-2-ol, chlorhexidine digluconate, chloroxylenol, triclosan, triclocarban, benzethonium chloride, methylbenzethonium chloride and benzalkonium chloride,
wherein the preparation additionally comprises or consists of
c) an antimicrobial active amount of one or more compounds selected from the group consisting of mecetroniumetil sulfate, undecyleneamidopropyltrimonium methosulfate, (ethylendioxy)dimethanol, benzyl-C12-18-alkyldimethylammoniumchloride, didecyldimethylammonium chloride, N,N-didecyl-N-methyl-poly(oxethyl)ammonium propionate, N-(3-aminopropyl)-N-dodecylpropan-1,3-diamin, N-dodecylpropan-1,3-diamin, N-(3-aminopropyl)-N-dodecylpropan-1,3-diamin, clorofen, 2-biphenyl-2-ol, chlorocresol, hydrogen peroxide, acetic acid, peracetic acid, glutaral and formaldehyde,
wherein the total amount of 1,2-decanediol of formula 1 in the preparation is in the range of from 0,1 to 2 wt.-% based on the weight of the preparation.

2. Preparation according to claim 1, wherein the preparation is in the form of an alcoholic, glycolic and/or aqueous cleansing solution.

3. Preparation according to claim 1, wherein the preparation is in the form of a disinfectant liquid or solid soap.

4. Preparation according to any of claims 1 to 3, wherein the preparation is in the form of an oil in water or water in oil emulsion.

5. Preparation according to any of claims 1 to 4, wherein constituent a) and/or one or more compounds selected from constituent b) are present in the finished preparation in an amount which is capable of disinfecting skin surface, preferably hand surface.

6. Preparation according to any of claims 1 to 5, wherein constituent a) and/or one or more compounds selected from constituent b) are present in the finished preparation in an amount which is capable of disinfecting technical surfaces.

7. Use of a preparation according to any of claims 1 to 6 as an antimicrobial preparation for technical surfaces.

8. Pharmaceutical product preparation according to any of claims 1 to 6 for use in a method for disinfection of skin, preferably for disinfection of hand surface comprising or consisting of the step:
a) application of a pharmaceutical product preparation according to any of claims 1 to 6 to skin surface, preferably to hand surface.

9. Method for disinfection of technical surfaces, comprising or consisting of the step:
a) application of a preparation according to any of claims 1 to 6 to technical surfaces.

10. Process for the production of a cosmetic and/or pharmaceutical preparation for disinfection of skin, preferably for disinfection of hand surface, and/or a household product preparation for disinfection of technical surfaces comprising or consisting of the following steps:
a) providing 1,2-decanediol of formula 1: 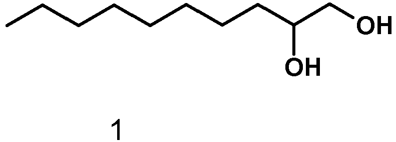
b) providing one or more compounds selected from the group of ethanol, propan-1-ol, propan-2-ol, chlorhexidine digluconate, chloroxylenol, triclosan, triclocarban, benzethonium chloride, methylbenzethonium chloride and benzalkonium chloride,
d) providing one or more compounds selected from the group consisting of mecetroniumetil sulfate, undecyleneamidopropyltrimonium methosulfate, (ethylendioxy)dimethanol, benzyl-C12-18-alkyldimethylammoniumchloride, didecyldimethylammonium chloride, N,N-didecyl-N-methyl-poly(oxethyl)ammonium propionate, N-(3-aminopropyl)-N-dodecylpropan-1,3-diamin, N-dodecylpropan-1,3-diamin, N-(3-aminopropyl)-N-dodecylpropan-1,3-diamin, clorofen, 2-biphenyl-2-ol, chlorocresol, hydrogen peroxide, acetic acid, peracetic acid, glutaral and formaldehyde, and
e) mixing one or more compounds provided in step d) with 1,2-decanediol provided in step a) and one or more compounds provided in step b) to form a preparation according to claims 1 to 6.

## Patentansprüche

1. Kosmetische, pharmazeutische und/oder Haushaltsprodukt-Zubereitung, enthaltend eine Mischung enthaltend oder bestehend aus
(a) einert antimikrobiell wirksame Menge von 1,2-Decandiol der Formel (I) 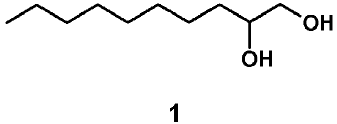 und
b) einer antimikrobiell wirksame Menge einer oder mehrerer Komponenten ausgewählt aus der Gruppe, die gebildet wird von Mecetroniumethylsulfat, Undecylenamidopropyltrimonium Methosulfat, (Ethylendioxy)dimethanol, Benzyl-C12-18-alkyldimethylammoniumchlorid, Didecyldimethylammoniumchlorid, N,N-Didecyl-N-methyl-poly(oxyethyl)ammoniumpropionat, N-(3-aminopropyl)-N-dodecylpro-pan-1,3-diamin, N-dodecylpropan-1,3-diamin, N-(3-aminopropyl)-N-dodecyl-propan-1,3-diamin, Chlorofen,2-Biphenyl-2-ol, Chlorocresol, Wasserstoffperoxid, Essigsäure, Peressigsäure, Glutaraldehyd und Formaldehyd,
wobei die Gesamtmenge des 1,2-Dodecandiols der Formel 1 in der Zubereitung im Bereich von 0,1 bis 2 Gew.-% basierend auf der Zubereitung liegt.

2. Zubereitung nach Anspruch 1, wobei die Zubereitung in Form einer alkoholischen, glykolischen und/oder wässrigen Reinigungslösung vorliegt.

3. Zubereitung nach Anspruch 1, wobei die Zubereitung in Form einer Desinfektionslösung oder eines Seifenstücks vorliegt.

4. Zubereitung nach jedem der Ansprüche 1 bis 3, wobei die Zubereitung in Form einer Ölin-Wasser oder Wasser-in-Öl Emulsion vorliegt.

5. Zubereitung nach jedem der Ansprüche 1 bis 4, wobei die Komponente (a) und/oder eine oder mehrere Stoffe der Komponente (b) in der Endzubereitung in einer ausreichenden Menge zugegen sind, um die Haut, vorzugsweise die Handflächen zu desinfizieren.

6. Zubereitung nach jedem der Ansprüche 1 bis 5, wobei die Komponente (a) und/oder eine oder mehrere Stoffe der Komponente (b) in der Endzubereitung in einer ausreichenden Menge zugegen sind, um technische Oberflächen zu desinfizieren.

7. Verwendung einer Zubereitung nach jedem der Ansprüche 1 bis 6 als antimikrobielle Zubereitung für technische Oberflächen.

8. Pharmazeutische Produktzubereitung nach jedem der Ansprüche 1 bis 6 zur Verwendung in einem Verfahren zur Desinfektion der Haut, vorzugsweise der Handfläche umfassend oder bestehend aus dem Schritt:
a) Auftragen einer pharmazeutischen Produktzubereitung nach jedem der Ansprüche 1 bis 6 auf die Hautoberfläche, insbesondere die Handfläche.

9. Verfahren zur Desinfektion von technischen Oberflächen, umfassend oder bestehend aus dem Schritt:
a) Auftragen einer Zubereitung nach jedem der Ansprüche 1 bis 6 auf eine technische Oberfläche.

10. Verfahren zur Herstellung einer kosmetischen und/oder pharmazeutischen Zubereitung zur Desinfektion der Haut, insbesondere zur Desinfektion der Handflächen und/oder einer Haushaltsproduktzubereitung zur Desinfektion von technischen Oberflächen enthaltend oder umfassend die folgenden Schritte:
a) Bereitstellung von 1,2-Decandiol der Formel 1 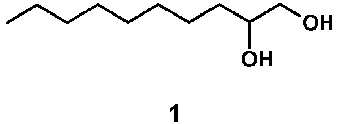
b) Bereitstellung einer oder mehrerer Komponenten ausgewählt aus der Gruppe Ethanol, Propan-1-ol, Propan-2-ol, Chlorhexidindigluconat, Chloroxylenol, Trichlosan, Triclocarban, Benzethoniumchlorid, Methylbenzethoniumchlorid und Benzalkoniumchlorid,
d) Bereitstellung einer oder mehrerer Komponenten ausgewählt aus der Gruppe, die gebildet wird von Mecetroniumethylsulfat, Undecylenamidopropyltrimonium Methosulfat, (Ethylendioxy)dimethanol, Benzyl-C12-18-alkyldimethylammonium-chlorid, Didecyldimethylammoniumchlorid, N,N-Didecyl-N-methyl-poly(oxyethyl)-ammoniumpropionat, N-(3-aminopropyl)-N-dodecylpropan-1,3-diamin, N-dode-cylpropan-1,3-diamin, N-(3-aminopropyl)-N-dodecylpropan-1,3-diamin, Chloro-fen,2-Biphenyl-2-ol, Chlorocresol, Wasserstoffperoxid, Essigsäure, Peressigsäure, Glutaraldehyd und Formaldehyd, und
e) Vermischen einer oder mehrerer Komponenten, die in Schritt d) bereitgestellt wurden, mit 1,2-Dodecandiol bereitgestellt in Schritt a) und einer oder mehrerer Komponenten bereitgestellt in Schritt b) zur Herstellung einer Zubereitung nach den Ansprüchen 1 bis 6.

## Revendications

1. Préparation cosmétique, pharmaceutique et/ou de produit ménager, comprenant un mélange comprenant, ou étant constitué de,
a) une quantité active antimicrobienne de 1,2-décanediol de formule 1 : 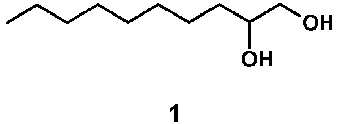 et
b) une quantité active antimicrobienne d'un ou plusieurs composés choisis dans le groupe constitué par l'éthanol, le propan-1-ol, le propan-2-ol, le digluconate de chlorhexidine, le chloroxylénol, le triclosan, le triclocarban, le chlorure de benzéthonium, le chlorure de méthylbenzéthonium et le chlorure de benzalkonium,
où la préparation, en outre, comprend, ou est constituée de,
c) une quantité active antimicrobienne d'un ou plusieurs composés choisis dans le groupe constitué par l'éthylsulfate de mécétronium, le méthosulfate d'undécylèneamidopropyltrimonium, le (éthylènedioxy)diméthanol, le chlorure de benzyl-C₁₂₋₁₈-alkyldiméthylammonium, le chlorure de didécyldiméthylammonium, le propionate de N,N-didécyl-N-méthyl-poly(oxoéthyl)ammonium, la N-(3-aminopropyl)-N-dodécylpropan-1,3-diamine, la N-dodécylpropan-1,3-diamine, la N-(3-aminopropyl)-N-dodécylpropan-1,3-diamine, le clorofène, le 2-biphényl-2-ol, le chlorocrésol, le peroxyde d'hydrogène, l'acide acétique, l'acide peracétique, le glutaraldéhyde et le formaldéhyde,
où la quantité totale de 1,2-décanediol de formule 1 dans la préparation se trouve dans la plage allant de 0,1 à 2% en poids sur la base du poids de la préparation.

2. Préparation selon la revendication 1, où la préparation se trouve sous la forme d'une solution de nettoyage alcoolique, glycolique et/ou aqueuse.

3. Préparation selon la revendication 1, où la préparation se trouve sous la forme d'un savon solide ou liquide désinfectant.

4. Préparation selon l'une quelconque des revendications 1 à 3, où la préparation se trouve sous la forme d'une émulsion huile-dans-eau ou eau-dans-huile.

5. Préparation selon l'une quelconque des revendications 1 à 4, où le constituant a) et/ou un ou plusieurs composés choisis parmi le constituant b) sont présents dans la préparation finie selon une quantité qui est capable de désinfecter la surface de la peau, préférablement la surface des mains.

6. Préparation selon l'une quelconque des revendications 1 à 5, où le constituant a) et/ou un ou plusieurs composés choisis parmi le constituant b) sont présents dans la préparation finie selon une quantité qui est capable de désinfecter des surfaces techniques.

7. Utilisation d'une préparation selon l'une quelconque des revendications 1 à 6, comme préparation antimicrobienne pour des surfaces techniques.

8. Préparation de produit pharmaceutique selon l'une quelconque des revendications 1 à 6, pour une utilisation dans une méthode de désinfection de la peau, préférablement de désinfection de la surface des mains, comprenant ou consistant en l'étape
a) d'application d'une préparation de produit pharmaceutique selon l'une quelconque des revendications 1 à 6 à la surface de la peau, préférablement la surface des mains.

9. Méthode de désinfection de surfaces techniques, comprenant ou consistant en l'étape
a) d'application d'une préparation selon l'une quelconque des revendications 1 à 6 à des surfaces techniques.

10. Procédé de production d'une préparation cosmétique et/ou pharmaceutique destinée à la désinfection de la peau, préférablement la désinfection de la surface des mains, et/ou d'une préparation de produit ménager destinée à la désinfection de surfaces techniques, comprenant ou consistant en les étapes suivantes
a) la fourniture de 1,2-décanediol de formule 1 : 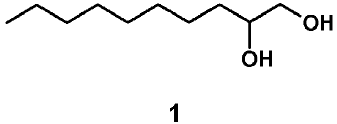
b) la fourniture d'un ou plusieurs composés choisis dans le groupe constitué par l'éthanol, le propan-1-ol, le propan-2-ol, le digluconate de chlorhexidine, le chloroxylénol, le triclosan, le triclocarban, le chlorure de benzéthonium, le chlorure de méthylbenzéthonium et le chlorure de benzalkonium,
d) la fourniture d'un ou plusieurs composés choisis dans le groupe constitué par l'éthylsulfate de mécétronium, le méthosulfate d'undécylèneamidopropyl-trimonium, le (éthylènedioxy)diméthanol, le chlorure de benzyl-C₁₂₋₁₈-alkyldiméthylammonium, le chlorure de didécyldiméthylammonium, le propionate de N,N-didécyl-N-méthyl-poly(oxoéthyl)ammonium, la N-(3-aminopropyl)-N-dodécylpropan-1,3-diamine, la N-dodécylpropan-1,3-diamine, la N-(3-aminopropyl)-N-dodécylpropan-1,3-diamine, le clorofène, le 2-biphényl-2-ol, le chlorocrésol, le peroxyde d'hydrogène, l'acide acétique, l'acide peracétique, le glutaraldéhyde et le formaldéhyde, et
e) le mélange d'un ou plusieurs composés fournis dans l'étape d) avec le 1,2-décanediol fourni dans l'étape a) et un ou plusieurs composés fournis dans l'étape b), afin de former une préparation selon les revendications 1 à 6.
